# FASCICULE DE BREVET EUROPEEN

(11) **EP 2 755 579 B1**
(45) Date de publication et mention de la délivrance du brevet: **06.04.2016**
(21) Numéro de dépôt: 12753998.9
(22) Date de dépôt: 04.09.2012
(51) Int. Cl.: A61B 17/16

(54) **OUTIL CHIRURGICAL POUR L'ALÉSAGE DU CANAL DIAPHYSAIRE D'OS LONGS**
CHIRURGISCHES WERKZEUG ZUM BOHREN IN DER MARKHÖHLE VON RÖHRENKNOCHEN
SURGICAL TOOL FOR DRILLING A BORE IN THE DIAPHYSEAL CAVITY OF LONG BONES

(30) Priorité: 16.09.2011 CH 15362011
(43) Date de publication de la demande: 23.07.2014
(73) Titulaire: Chirmat Sàrl, 1870 Monthey (CH)
(72) Inventeur: ARLETTAZ, Yvan, CH-1870 Monthey (CH); BONJOUR, Christian, CH-1268 Begnins (CH)
(74) Mandataire: P&TS SA (AG, Ltd.)
(86) Numéro de dépôt international: PCT/EP2012/067240
(87) Numéro de publication internationale: WO 2013/037673

(56) Documents cités:
- EP-A1- 2 335 617
- EP-A2- 0 648 477
- EP-A2- 1 987 785
- US-A1- 2004 236 339
- US-A1- 2006 047 309
- US-A1- 2006 064 164
- US-A1- 2010 262 149

## Description

### Domaine technique

La présente invention concerne une tête d'alésage pour l'élargissement du canal médullaire dans le traitement des fractures des os longs.

### Etat de la technique

L'enclouage centromédullaire est une des techniques thérapeutiques les plus utilisées dans le traitement des fractures et dans la reconstruction des os longs, notamment lors de fracture du corps du fémur, mais cette technique peut être appliquée aussi à d'autres os longs tels que l'humérus ou le tibia. La technique de l'alésage centromédullaire consiste en l'élargissement du canal diaphysaire avec une tête d'alésage, ou une série de têtes d'alésage de diamètre croissant, montée sur un arbre d'entraînement souple. L'opération d'alésage est préliminaire à l'insertion d'un clou ou broche métallique dans le canal diaphysaire, afin de réduire et stabiliser la fracture et, en même temps, soulager la charge de compression sur l'os.

L'alésage centromédullaire, réalisant un calibrage au diamètre souhaité du canal diaphysaire, améliore l'adaptation et l'adhésion du clou à la paroi du canal médullaire et réduit considérablement le risque de coincement du clou ou d'éclatement du corps de l'os long lors de l'insertion de l'implant.

En plus des avantages mécaniques suscités, Cette technique présente aussi des importants avantages sur le plan biologique et clinique : notamment elle peut transporter des particules osseuses dans le site de la fracture, avec un effet équivalent à une autogreffe ce qui participe au processus de guérison.

Pour assurer que l'alésage soit parfaitement concentrique au canal médullaire, on insère préalablement une tige de guidage (aussi connue sous le nom de tige de Kirschner) jusqu'à la métaphyse distale par rapport à fracture. La tête d'alésage et son arbre d'entrainement ont un trou axial lui permettant de glisser concentriquement sur la tige de guidage. Les tiges de guidage sont préférablement équipées d'une olive ou une protubérance à l'extrémité distale pour récupérer, si nécessaire, une tête d'alésage bloquée ou endommagé.

Cette technique est conseillée de par ses indéniables avantages cliniques. Elle comporte également certains défauts liés notamment aux pressions et aux échauffements qui se produisent à l'intérieur du canal médullaire lors de l'alésage et qui pourraient provoquer des embolies graisseuses. Une technique chirurgicale appropriée comportant des vitesses de coupe et d'avancement modérées et limitant l'épaisseur enlevée à 0.2 à 0.5 mm (au rayon) par passage, permettent de réduire ces risques, sans toutefois les éliminer complètement.

On connaît des outils spécifiques pour l'alésage du canal médullaire, par exemple les alésoirs décrits par les documents EP0648477, EP2335617, EP1987785. Le document US2004236339 décrit un dispositif de guidage pour l'alésage centromédullaire, tandis que US2006064164 divulgue des fraises orthopédiques utilisées préalablement à la pose d'un implant lors d'une fracture du col du fémur. Ces dispositifs sont toutefois coûteux et doivent être réutilisés. Ils sont cependant difficiles à nettoyer et à stériliser, particulièrement en milieu hospitalier. Leur usage est délicat en raison des surpressions qu'ils engendrent dans l'os lors de l'opération, et de l'échauffement par friction qu'ils provoquent, particulièrement si leurs tranchants ne sont pas parfaitement affutés. US2010262149 et US2006047309 décrivent des instruments médicaux réalisés en technologie MIM (Moulage à Injection de Métal).

### Bref résumé de l'invention

Un but de la présente invention est de proposer une tête d'alésage exempte des limitations des outils connus, et notamment une tête d'alésage qui permet de minimiser les effets de surpression et d'échauffement lors de l'élargissement du canal médullaire.

Un autre but de l'invention est de proposer une tête d'alésage plus économique que les dispositifs connus, autorisant son usage unique.

Selon l'invention, ces buts sont atteints notamment au moyen de l'objet des revendications annexées, et notamment par un outil chirurgical pour l'alésage du canal diaphysaire d'os longs comprenant : une âme centrale et une douille périphérique coaxiale avec ladite âme centrale et connectée par une pluralité d'ailettes définissant une pluralité d'ouvertures axiales reliant une face antérieure de l'outil avec une face postérieure de l'outil, et des dents sur la surface extérieure de ladite douille périphérique.

### Brève description des figures

Des exemples de mise en oeuvre de l'invention sont indiqués dans la description illustrée par les figures annexées dans lesquelles :

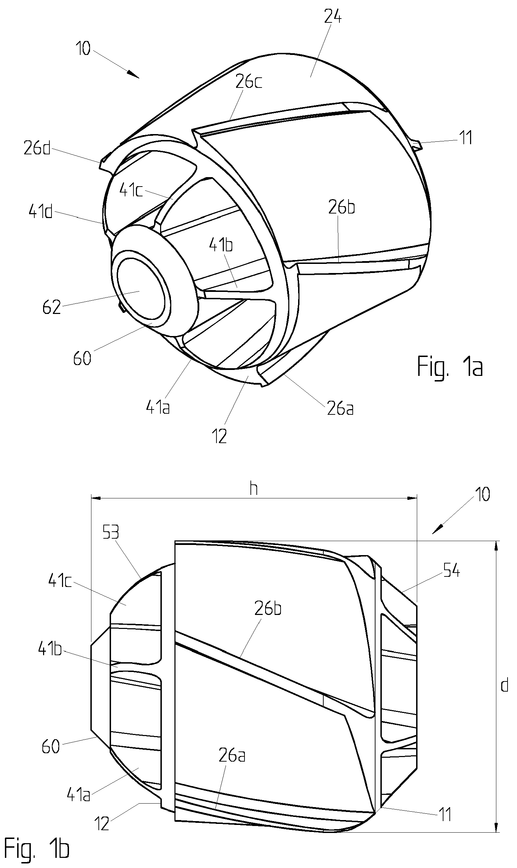

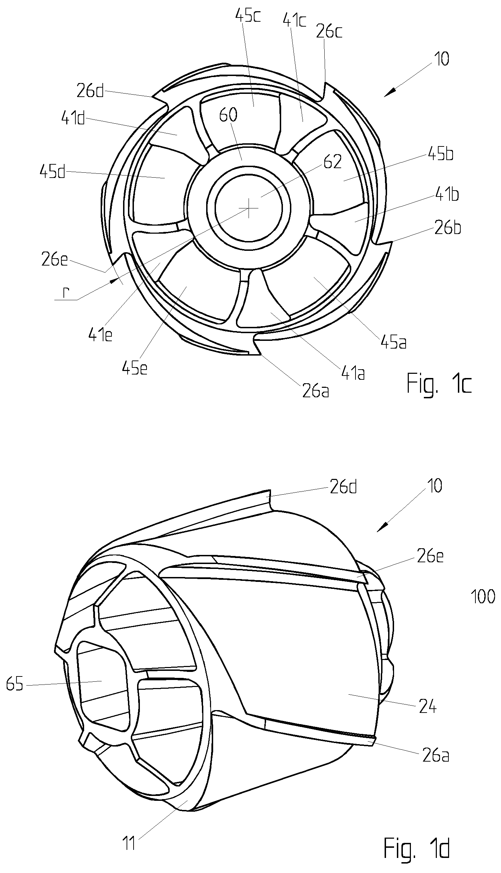

- Les figures 1a et 1d illustrent une tête d'alésage selon un aspect de l'invention vu par la face avant et, respectivement, par la face arrière;
- Les figures 1 b et 1c illustrent la tête d'alésage des figures 1a et 1d vue de côté et de face.
- La Figure 2 illustre les angles caractéristiques de la tête d'alésage des figures 1a-1d ;
- La Figure 3 illustre un possible système de connexion entre un alésoir selon l'invention et un arbre d'entraînement.

### Exemple(s) de mode de réalisation de l'invention

Avec référence aux figures de 1a à 1d, un alésoir selon un aspect de l'invention comporte une douille cylindrique ou tronc-conique 24 portant des dents hélicoïdales 26a-26e sur sa surface extérieure. Le rayon r de la tête d'alésage 10 est choisi en fonction des dimensions de l'os à traiter et du diamètre de canal que l'on souhaite obtenir. Préférablement, les têtes d'alésage sont réalisés en série avec des dimensions progressivement croissantes, par exemple avec une progression du rayon de 0,2-0,5 mm entre deux éléments successifs. Cela permet au chirurgien d'effectuer l'alésage en plusieurs passages, enlevant une modique quantité d'os cortical à chaque passage. Dans le cas d'une fracture du corps du fémur on préconise, d'une manière générale, de porter le canal médullaire à un diamètre final entre 11 et 14 mm avant d'insérer le clou, mais plusieurs facteurs peuvent influencer ces dimensions, la dimension du patient, la nature de la fracture et le type de clou entre autres.

La hauteur de la tête d'alésage mesurée selon l'axe de rotation est choisie en relation avec ses dimensions latérales, étant préférablement comprise entre 5 et 20 mm en fonction du diamètre de la tête d'alésage.

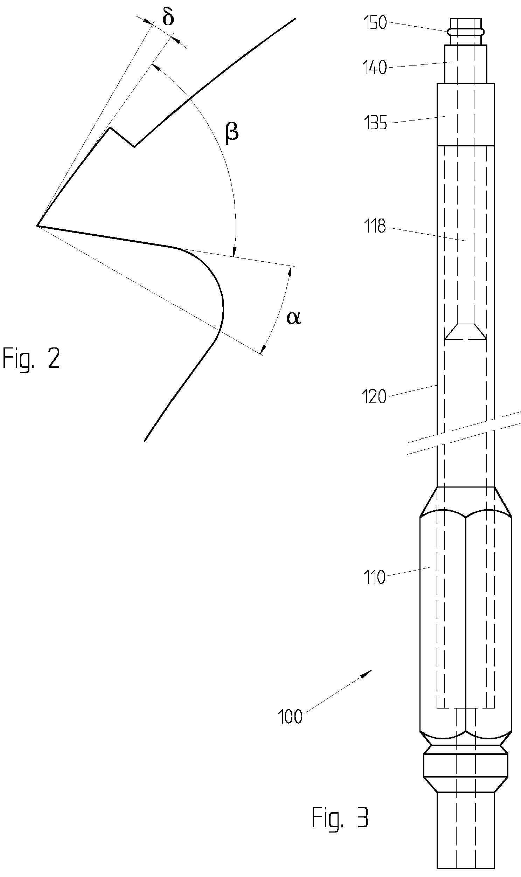

La figure 2 montre le profil d'une des dents hélicoïdales 26a-e . Préférablement, les dents ont un angle de coupe α important, pour réduire la force de taille, et aussi un angle de dépouille δ prononcé, afin de minimiser le frottement de l'arrière de la dent sur la partie usinée. L'angle de taillant β, déterminé par la relation α+β+δ=90°, est par conséquent modéré, mais pas au point d'affaiblir la dent. Des essais ont permis de conclure que des angles compris entre15 et 40 °, typiquement entre 20 et 30° pour α et entre 3 et 20°, typiquement entre 7 et 15° pour δ offrent une performance optimale. L'angle d'hélice des dents est préférablement compris entre 15° et 35°. Les têtes d'alésage chirurgicales sont généralement conçues pour tourner en sens horaire, et l'angle d'hélice est par conséquent à droite, de manière à extraire le matériau coupé vers l'arrière de l'outil.

Les figures se réfèrent au cas d'un outil à cinq dents, mais on voit bien que cela n'est pas une caractéristique limitative de l'invention. La tête d'alésage peut porter un nombre de dents différent, par exemple deux, trois, ou quatre, voire six ou plus.

Revenant maintenant à la figure 1, la tête d'alésage présente une âme 60 coaxiale avec la douille 24 avec un trou central 62 pour le passage de la tige de Kirschner. Dans ce mode de réalisation, le trou axial 62 de la face avant a une section ronde (Fig. 1a) dont le diamètre correspond à celui de la tige de Kirschner, tandis qu'au niveau de la face arrière la tête d'alésage présente par exemple un trou axial 65 de section polygonale (Fig. 1d) pour permettre la transmission du couple moteur depuis un arbre d'entrainement, comme on le verra plus bas. D'autres formes seraient également possibles dans le cadre de l'invention.

L'âme 60 et la douille 24 sont solidarisées rigidement par des ailettes radiales 41 a-41 e qui laissent ouverte une grande partie de la surface entre l'âme 60 et la douille 24. Dans les dessins, l'alésoir présente cinq ailettes et quatre ouvertures axiales 45a-45e, mais cela n'est pas une caractéristique nécessaire de l'invention, les ailettes et ouvertures pouvant être en un nombre quelconque.

L'effet des ouvertures axiales 45a-e est de permettre le passage de la moelle osseuse de l'avant à l'arrière de l'outil lorsque celui-ci avance dans le canal diaphysaire, allégeant ainsi la surpression dans le canal et réduisant le risque d'embolie graisseuse ou d'éclatement.

On peut assimiler le canal diaphysaire à un cylindre de diamètre constant avec une extrémité distale poreuse rempli d'un liquide de viscosité élevé, incompressible aux effets pratiques, et la tête d'alésage par un disque qui est poussé vers l'intérieur depuis l'extrémité proximale. L'écoulement du matériau interne est déterminé par les équations de continuité et de conservation de la masse. Trois cas sont intéressants:
- si le disque est plein et le jeu entre le disque et la paroi est très faible, la pression va fortement augmenter dans le cylindre; une partie du liquide visqueux sortira par l'extrémité poreuse et une autre par le jeu entre la tête et la paroi : la surpression favorise le passage d'une partie du fluide dans la circulation sanguine, et par conséquent le risque d'embolie graisseuse est accru.
- si le disque est plein et le jeu entre le disque et la paroi est important, la pression va augmenter, mais ne sera pas suffisante pour provoquer une percolation du liquide visqueux à travers l'extrémité poreuse, le déplacement du piston sera difficile et provoquera un échauffement par frottement du liquide (rapport des vitesses dans le rapport des surfaces)
- si en revanche le disque est ajouré et présente des ouvertures axiales mettant en communication la face antérieure avec la face postérieure, et l'issue vers sa face arrière est dégagée, la pression n'augmentera quasiment pas, il n'y aura n'y percolation à l'extrémité poreuse, ni augmentation de la température car la majorité du liquide visqueux s'échappera par le centre du disque.

On voit donc comment la structure de la tête d'alésage de l'invention permet le passage de la moelle osseuse par les ouvertures 45a-45e, assurant ainsi un avancement plus facile et réduisant la pression à l'intérieur du canal diaphysaire. De cette manière, la tête d'alésage de l'invention simplifie l'acte chirurgical et réduit la probabilité d'embolie ou autres issues défavorables.

Selon une variante préférée de l'invention, les ailettes 41a-e ne sont pas contenues dans des plans axiaux, mais sont courbées ou inclinées de façon à favoriser l'extraction de la moelle vers la face arrière par la rotation de l'outil sans la comprimer, à la manière d'une vis tarière. La disposition inclinée ou courbée augmente également la rigidité de l'outil par rapport à une structure avec des ailettes planes.

Dans un mode de réalisation préféré, la face avant de la tête d'alésage n'est pas plane mais comporte une section convergente 53, par exemple de profil bombé ou tronc-conique, facilitant le centrage de l'outil dans le canal diaphysaire. La face arrière comporte préférablement elle aussi une section convergente 54, par exemple de profil bombé ou tronc-conique, avec le but de réduire le frottement de l'outil.

L'outil de l'invention est préférablement réalisé en acier chirurgical ou tout autre matériel idoine, et destiné préférablement à un usage unique, (mais pas exclusivement), de cette façon le chirurgien dispose, pour chaque opération d'alésage, d'un outil parfaitement affûté, ce qui réduit considérablement les efforts de coupe, le frottement et l'échauffement. Préférablement, il est réalisé avec la technique MIM (Métal Injection Molding, ou Moulage à Injection de Métal) afin de réduire son prix de revient.

Préférablement, l'âme centrale 60 a un faible diamètre, de façon à maximiser la section des ouvertures. Préférablement l'âme 60 aura un diamètre inférieur à 7,0 mm, plus préférablement égal ou inférieur à 5,0 mm; le diamètre de l'âme est en relation avec le diamètre de la tête d'alésage. L'arbre d'entraînement et le support destiné à solidariser la tête d'alésage avec l'arbre ont préférablement eux aussi des diamètres limités, afin de ne pas entraver l'écoulement de la moelle. On utilisera des supports avec un diamètre inférieur à 9,0 mm, préférablement inférieur à 8,0 mm; le diamètre de l'arbre d'entrainement est en relation avec l'os long alésé. La présente invention peut être utilisée avec plusieurs formes d'arbres d'entrainement et supports.

La tête d'alésage préférentiellement à usage unique de l'invention serait préférablement accouplé avec un arbre d'entrainement à usage unique en composite, comme par exemple les dispositifs décrits dans la demande de brevet International WO2011051260 au nom de la demanderesse et représentés dans la figure 3.

L'arbre d'entraînement 100 est entièrement traversé axialement par un canal axial 118 destiné à permettre le passage de la tige de Kirschner. A une extrémité, il présente un élément d'interface 110 pour l'accouplement avec un mandrin d'un moteur, par exemple un élément prismatique hexagonal, ou tout autre élément d'interface idoine. Le fût 120 de l'arbre d'entraînement est préférablement une tige souple réalisée avec un matériau composite comprenant des fibres de carbone et/ou de verre englobées en une matrice de résine contenant un élastomère. Cette forme de réalisation permet de proposer des arbres d'entraînement avec des caractéristiques d'élasticité et résistance optimales à un coût très avantageux. La tête d'alésage de l'invention, toutefois, est utilisable en combinaison avec n'importe quel type d'arbre de transmission : on pourrait par exemple l'accoupler avec des arbres de transmission métalliques classiques, ou avec des arbres de transmission en composite avec des caractéristiques différentes, sans sortir du cadre de l'invention.

La connexion entre l'arbre 100 et les têtes d'alésage est obtenue, dans ce mode de réalisation, par un élément de connexion 135 comportant un tenon 140 à section polygonale qui épouse la forme du trou axial 65 de la face arrière de la tête d'alésage. La stabilité en direction axiale est assurée, par exemple, par l'anneau élastique 150 qui s'engage en une gorge correspondante (non visible dans les figures) de la tête d'alésage.

Cette disposition permet un échange facile des têtes d'alésage et une excellente transmission du couple moteur tout en restant très compacte. L'invention toutefois n'est pas limitée au système de couplage décrit ci-dessus et représenté dans les figures, mais englobe également des tenons de n'importe quelle forme, par exemple en queue d'aronde, des clavettes, des couplages à vis, ou tout autre système de couplage approprié.

On peut ainsi proposer un assortiment complet de têtes d'alésage et un arbre de transmission sous forme de kit à usage unique, éliminant ainsi tous les problèmes de nettoyage et stérilisation des outils connus et offrant toujours des outils affûtés et parfaitement performants.

### Numéros de référence employés sur les figures

- α: angle de coupe
- β: angle de taillant
- δ: angle de dépouille
- 10: tête d'alésage
- 11: face arrière
- 12: face avant
- 24: douille
- 26a-e: dents
- 41a-e: ailettes
- 45a-e: ouvertures axiales
- 53: section convergente face avant
- 54: section convergente face arrière
- 60: âme
- 62: trou axial face avant
- 65: trou axial face arrière
- 100: arbre d'entrainement
- 110: interface moteur
- 118: canal axial
- 120: tige souple
- 135: élément de connexion
- 140: tenon à section polygonale
- 150: anneau élastique

## Revendications

1. Outil chirurgical (10) pour l'alésage du canal diaphysaire d'os longs, comprenant : une âme centrale (60) et une douille périphérique (24) coaxiale avec ladite âme centrale (60) et connectée par une pluralité d'ailettes (41a-41e) définissant une pluralité d'ouvertures axiales (45a-45d) reliant une face antérieure (12) de l'outil (10) avec une face postérieure (11) de l'outil (10), et des dents (26a-26e) sur la surface extérieure de ladite douille périphérique (24).

2. Outil chirurgical (10) selon l'une des revendications précédentes, dans lequel lesdites ailettes (41a-41e) présentent des surfaces courbes ou inclinées.

3. Outil chirurgical (10) selon l'une des revendications précédentes, dans lequel ladite âme (60) comporte un trou axial (62).

4. Outil chirurgical (10) selon l'une des revendications précédentes, dans lequel lesdites dents ont une disposition hélicoïdale.

5. Outil chirurgical (10) selon l'une des revendications précédentes, dans lequel ladite douille a une forme essentiellement cylindrique ou tronc-conique.

6. Combinaison d'au moins un outil chirurgical (10) selon l'une des revendications précédentes, et d'un arbre de transmission (100) comprenant une tige souple (120) en composite avec des fibres de carbone et/ou de verre englobés en une matrice polymérique comprenant un élastomère.

7. Outil chirurgical (10) selon l'une des revendications de 1 à 5, réalisé par Moulage à Injection de Métal.

## Patentansprüche

1. Chirurgisches Werkzeug (10) zum Bohren in der Markhöhle von Röhrenknochen, mit: einem zentralen Kern (60) und einer peripheren Hülse (24), welche koaxial zum besagten zentralen Kern (60) angeordnet und durch eine Vielzahl von Flügeln (41a-41e) verbunden ist, die eine Vielzahl von axialen Öffnungen (45a-45d) definieren, welche eine Vorderfläche (12) des Werkzeugs (10) mit einer Rückfläche (11) des Werkzeugs (10) verbinden, und Zähnen (26a-26e) auf der Aussenfläche der besagten peripheren Hülse (24).

2. Chirurgisches Werkzeug (10) gemäss einem der vorhergehenden Ansprüche, worin die besagten Flügel (41a-41e) gebogene oder geneigte Flächen aufweisen.

3. Chirurgisches Werkzeug (10) gemäss einem der vorhergehenden Ansprüche, worin der besagte Kern (60) eine axiale Bohrung (62) aufweist.

4. Chirurgisches Werkzeug (10) gemäss einem der vorhergehenden Ansprüche, worin die besagten Zähne auf spiralförmige Weise angeordnet sind.

5. Chirurgisches Werkzeug (10) gemäss einem der vorhergehenden Ansprüche, worin die besagte Hülse im Wesentlichen eine zylindrische oder kegelstumpfförmige Form aufweist.

6. Kombination von mindestens einem chirurgischen Werkzeug (10) gemäss einem der vorhergehenden Ansprüche und einer Antriebswelle (100) mit einem Biegestab (120) aus Verbundwerkstoff mit in einer Polymermatrix mit einem Elastomer eingekapselten Kohl- und/oder Glasfasern.

7. Verwendung der Metallspritzgiessen-Technologie (Metal Injection Moulding MIM) zur Herstellung des chirurgischen Werkzeugs gemäss einem der Ansprüche 1 bis 6.

## Claims

1. Surgical tool (10) for reaming the diaphyseal canal of long bones, comprising: a central core (60) and a peripheral bushing (24) that is coaxial to said central core (60) and connected via a plurality of vanes (41a-41 e) defining a plurality of axial openings (45a-45d) connecting a front surface (12) of the tool (10) to a rear surface (11) of the tool (10), and teeth (26a-26e) on the outer surface of said peripheral bushing (24).

2. Surgical tool (10) according to one of the preceding claims, wherein said vanes (41a-41e) have curved or inclined surfaces.

3. Surgical tool (10) according to one of the preceding claims, wherein said core (60) comprises an axial hole (62).

4. Surgical tool (10) according to one of the preceding claims, wherein said teeth are arranged in a helical fashion.

5. Surgical tool (10) according to one of the preceding claims, wherein said bushing has an essentially cylindrical or truncated-cone shape.

6. Combination of at least one surgical tool (10) according to one of the preceding claims and of a transmission shaft (100) having a flexible rod (120) of composite with carbon and/or glass fibres encapsulated in a polymeric matrix comprising an elastomer.

7. Use of the metal injection moulding (MIM) technique for making the surgical tool according to one of the claims 1 to 6.
